(19) **Europäisches Patentamt**
**European Patent Office**
**Office européen des brevets**

(11) **EP 3 216 598 B1**

(12) **EUROPEAN PATENT SPECIFICATION**

(45) Date of publication and mention
of the grant of the patent:
**29.01.2020 Bulletin 2020/05**

(21) Application number: **17159704.0**

(22) Date of filing: **07.03.2017**

(51) Int Cl.:
*B32B 18/00* *(2006.01)*       *C04B 35/111* *(2006.01)*
*C04B 35/16* *(2006.01)*       *C04B 35/486* *(2006.01)*
*C04B 35/626* *(2006.01)*       *C04B 35/634* *(2006.01)*
*B33Y 80/00* *(2015.01)*       *B33Y 70/00* *(2020.01)*
*B33Y 10/00* *(2015.01)*       *B28B 1/00* *(2006.01)*
*A61K 6/00* *(2020.01)*       *A61K 6/02* *(2006.01)*
*A61K 6/027* *(2006.01)*

(54) **MEDICAL DEVICE, METHOD FOR PRODUCING MEDICAL DEVICE**

MEDIZINISCHE VORRICHTUNG, VERFAHREN ZUR HERSTELLUNG EINER MEDIZINISCHEN
VORRICHTUNG

DISPOSITIF MÉDICAL, PROCÉDÉ DE PRODUCTION DE DISPOSITIF MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.03.2016 JP 2016046115**

(43) Date of publication of application:
**13.09.2017 Bulletin 2017/37**

(73) Proprietor: **Ricoh Company, Ltd.
Tokyo 143-8555 (JP)**

(72) Inventors:
• **WATANABE, Masaki**
**Ohta-ku, Tokyo 143-8555 (JP)**
• **SAKURAI, Yoichi**
**Ohta-ku, Tokyo 143-8555 (JP)**
• **SAITO, Takuya**
**Ohta-ku, Tokyo 143-8555 (JP)**
• **NIIMI, Tatsuya**
**Ohta-ku, Tokyo 143-8555 (JP)**

(74) Representative: **Lamb, Martin John Carstairs
Marks & Clerk LLP
15 Fetter Lane
London EC4A 1BW (GB)**

(56) References cited:
**WO-A1-2014/018325       US-A- 5 697 043
US-A1- 2010 145 469       US-A1- 2012 315 600
US-A1- 2015 259 247**

• **KUMAR ALOK ET AL: "Low temperature additive
manufacturing of three dimensional scaffolds for
bone-tissue engineering applications:
Processing related challenges and property
assessment", MATERIALS SCIENCE AND
ENGINEERING: R: REPORTS, ELSEVIER,
AMSTERDAM, NL, vol. 103, 4 March 2016
(2016-03-04), pages 1-39, XP029512075, ISSN:
0927-796X, DOI: 10.1016/J.MSER.2016.01.001**

**Description**

BACKGROUND OF THE INVENTION

Field of the Invention

The present invention relates to a medical device, and a method for producing a medical device.

Description of the Related Art

Hitherto, metal materials such as titanium, titanium alloys, and cobalt-chromium alloys and ceramics such as alumina, zirconia, and calcium phosphate have been used as materials for medical devices such as bone plates and dental prosthesis used for compensating for defective portions in, for example, orthopedics and oral surgery.

These medical devices are often dense bodies in terms of, for example, mechanical strength and discoloration. However, the dense bodies have problems that an enormous time is needed to adhere the dense bodies to soft tissues, particularly in highly invasive cases.

In order to solve this problem, in the production process of the medical devices, the surfaces of the medical devices, which are dense bodies, are roughened to promote soft tissues to adhere to the surfaces, or only the surfaces of the medical devices are made porous so it is easy for soft tissues to come into the pores of the porous surfaces to promote adhesion. However, in order to intentionally roughen the surfaces of the medical devices, there is a need for firstly machining the medical devices into desired shapes and then subjecting the medical devices to surface treatments such as etching. Complicated processes are also needed to make the surfaces porous. This increases the number of steps needed, resulting in a problem that the production process lags behind. This problem leads to prolongation of the defective states of patients' hard tissues.

The prolongation of the defective states of the hard tissues may foster invasion of soft tissues into the defective portions, involves risks of reoperations in some cases, and increases physical burdens on the patients. Hence, for producing the medical devices, it is demanded that desired shapes in terms of also surface profiles can be formed with only one step if possible and can be quickly delivered to the patients.

Hence, in recent years, many kinds of modeling processes have been generated along with the development of 3D printers, and there have been proposed methods for producing objects having rough surfaces by powder lamination modeling methods (see, e.g., Japanese Translation of PCT International Application Publication No. JP-T-2003-531034 and Japanese Unexamined Patent Application Publication No. 2011-21218. US 2012/315600 A1 discloses a ceramic dental implant.

The present invention has an object to provide a medical device that can be produced easily and efficiently and has a good dimensional accuracy and excellent adhesiveness with soft tissues.

SUMMARY OF THE INVENTION

According to one aspect of the present invention, a medical device includes a porous portion and a dense portion. An arithmetic average roughness of a surface of the porous portion is 2.0 $\mu$m or greater but 10 $\mu$m or less. An arithmetic average roughness of a surface of the dense portion is less than 2.0 $\mu$m. The porous portion has a voidage of 5% or greater. The dense portion has a voidage of less than 5%. The porous portion is a surface portion which contacts the soft tissue. The dense portion is a portion which does not contact the soft tissue. The porous portion and the dense portion comprise ceramic particles. The medical device is an artificial product intended for compensating for defective portions of hard tissues.

The present invention can provide a medical device that can be produced easily and efficiently and has a good dimensional accuracy and excellent adhesiveness with soft tissues.

BRIEF DESCRIPTION OF THE DRAWINGS

FIG. 1 is a schematic diagram illustrating an example of a medical device producing apparatus and

FIG. 2 is a schematic diagram illustrating another example of a medical device producing apparatus.

DESCRIPTION OF THE EMBODIMENTS

(Medical device)

A medical device of the present invention includes a porous portion and a dense portion. An arithmetic average roughness of a surface of the porous portion is 2.0 $\mu$m or greater but 10 $\mu$m or less. An arithmetic average roughness of a surface of the dense portion is less than 2.0 $\mu$m. The medical device further includes other portions as needed.

The medical device of the present invention is based on a problem that objects produced by existing powder lamination modeling methods can be provided with rough surfaces but cannot invite favorable adhesion of soft tissues if the surface roughness is increased in an uncontrolled manner, so there is a need for controlling the surface roughness to be close, to some degree, to a desired roughness.

The soft tissues refer to connective tissues such as tendon, ligament, fascia, skin, gingiva, and adipose tissue (except bone tissue), blood vessel, striated muscle, smooth muscle, peripheral nervous tissue (ganglion and nerve fiber), and cartilage.

The present inventors have found the following.

When the material of the medical device is metal particles or ceramic particles, powder lamination modeling methods are often employed considering the properties of the particles. In the case of the powder lamination modeling methods, the particles need to have some degree of particle diameter, lest fluidity of the powder should be lost due to influence of interactions between the particles and the powder cannot be conveyed. Furthermore, because the particles have some degree of the particle diameter, control at levels equal to or less than the particle diameter is unavailable. Meanwhile, in order to produce a medical device having a surface roughness (Ra) of a submicron order, at which it is said that cells can easily adhere to the surface, the particle diameter of the particles to be laminated needs to be small. Hence, it is found preferable to build up a process that can overcome the trade-off relationship between particle diameter and fluidity.

<Porous portion>

The porous portion may be appropriately selected depending on the intended purpose. The porous portion may be a portion of the medical device or most of the medical device. Of these portions, the porous portion is a surface portion to contact the soft tissue. The porous portion refers to a portion having a voidage of 5% or greater. The voidage can be calculated according a formula below.

$$\text{Voidage } (\%) = [(\text{true density} - \text{tap density})/\text{true density}] \times 100$$

The true density and the tap density in the formula can be measured with, for example, a powder characteristics tester (instrument name: POWDER TESTER (registered trademark) PT-X, available from Hosokawa Micron Group).

The porous portion is not particularly limited. For example, the diameter of the voids of the porous portion may be appropriately selected depending on the intended purpose.

The voidage of the porous portion is preferably 5% or greater but 80% or less and more preferably 10% or greater but 50% or less.

The diameter of the voids of the porous portion is not particularly limited, may be appropriately selected depending on the intended purpose, and is preferably 5 $\mu$m or greater but 1,000 $\mu$m or less and more preferably 10 $\mu$m or greater but 100 $\mu$m or less.

The arithmetic average roughness (Ra) of the surface of the porous portion is 2 $\mu$m or greater but 20 $\mu$m or less and preferably 2 $\mu$m or greater but 10 $\mu$m or less. When the arithmetic average roughness (Ra) of the surface of the porous portion is 2 $\mu$m or greater but 20 $\mu$m or less, a cell adhere sufficiently to the surface and the surface is more likely to be intertwined with a soft tissue. The arithmetic average roughness (Ra) can be measured according to JIS B 0601:2013.

It is preferable that the porous portion contain at least any one of a cell and a growth factor.

<<Cell and growth factor>>

The cell and the growth factor promote adhesion between the medical device and the soft tissue.

Generally, there are cases that adhesion of the cell is promoted by the presence of an adhesion factor. For example, when a cell is delivered to a cell adhesion factor such as fibronectin, the cell is likely to adhere to the site and to be kept there. Furthermore, the cell may promote osteogenesis when the cell is provided with an osteogenic factor that may activate an osteoblast or an osteoclast. That is, it is estimated that such a cell and a growth factor, when delivered to a specific site, enable the surface of the medical device, which is an artifact, to adhere more quickly to a soft tissue.

For example, for jaw lift, a basket-shaped device may be attached to a patient who does not have an alveolar ridge having a thickness enough to be implanted with a dental implant. In this case, the object may be entirely roughened by control during 3D modeling, and, for example, an osteogenic factor BMP and an osteoblast may be discharged from inkjet nozzles and delivered to specific sites in the object to be implanted in the object. It is estimated that this progresses jawbone formation more quickly and can shorten the time taken before implanting.

Furthermore, surfaces of a post crown may be made dense at the portions externally exposed and may be made rough at a portion to contact a gingiva, and a gingival fibroblast and a cell adhesion factor may be imparted to the rough portion. It is estimated that this allows a periodontal pocket to be filled during an initial period and to be less likely to catch an infectious disease.

The cell is not particularly limited and may be appropriately selected depending on the intended purpose so long as the cell is suitable for the site to be implanted with the medical device. When the medical device is used as a post crown, examples of the cell include a gingival fibroblast and a gingival epithelial progenitor cell. When the medical device is used as a bone plate, examples of the cell include an osteoblast and an osteoclast.

The growth factor is not particularly limited and may be appropriately selected depending on the intended purpose so long as the growth factor is suitable for the site to be implanted with the medical device. In the case of an object for jaw lift, examples of the growth factor include an osteogenic factor (BMP). In the case of an object for promoting adhesion with a cell, examples of the growth factor include cell adhesion factors such as cadherin, fibronectin, laminin, and vitronectin.

It is preferable to make the surface of the medical device contain at least any one of the cell and the growth factor, by discharging at least any one of a cell dispersion liquid in which the cell is dispersed and a growth factor-containing liquid containing the growth factor from an inkjet head.

The portion to contain the cell and the growth factor is preferably the porous portion of the medical device.

<Dense portion>

The dense portion may be appropriately selected depending on the intended purpose. The dense portion may be a portion of the medical device or most of the medical device. Of these portions, the dense portion is a portion not to contact the soft tissue. The dense portion refers to a portion having a voidage of less than 5%. The voidage can be calculated according a formula below.

$$\text{Voidage (\%)} = [(\text{true density}-\text{tap density})/\text{true density}]\times100$$

The true density and the tap density in the formula can be measured with a powder characteristics tester (instrument name: POWDER TESTER (registered trademark) PT-X, available from Hosokawa Micron Group).

The dense portion is not particularly limited. For example,

The voidage of the dense portion is preferably 3% or less.

The arithmetic average roughness (Ra) of the surface of the dense portion is less than 2 $\mu$m and preferably less than 1 $\mu$m. When the arithmetic average roughness (Ra) of the surface of the dense portion is less than 2 $\mu$m, the cell tends not to adhere to the surface, so formation of a biofilm on the surface can be prevented and the surface is less likely to be intertwined with the soft tissue. When the dense portion is used as an exposed portion of an artificial tooth, the dense portion can be prevented from discoloration. The arithmetic average roughness (Ra) can be measured according to JIS B 0601:2013.

The medical device is not particularly limited, may be appropriately selected depending on the intended purpose, and is preferably a laminated object.

The laminated object can be produced according to a method for producing a medical device of the present invention described below and using a medical device producing apparatus described below.

The laminated object is not particularly limited and may be appropriately selected depending on the intended purpose so long as the laminated object is formed by lamination. Examples of the laminated object include a laminated object formed of a ceramic material.

The ceramic material is not particularly limited and may be appropriately selected. It is preferable that ceramic material contain at least one selected from the group consisting of zirconia, alumina, and lithium disilicate.

The medical device refers to artificial products on the whole intended for compensating for defective portions of hard tissues.

The medical device is not particularly limited and may be appropriately selected depending on the intended purpose so long as the medical device is used for medical purposes. Examples of the medical device include dental prostheses, bone plates, artificial bones, and artificial joints.

The dental prostheses refer to artificial teeth created in place of natural teeth lost due to, for example, dental caries, external injuries, and periodontal disease for recovering the functions of the natural teeth. Examples of the dental prostheses include implants, plate dentures, and post crowns.

(Method for producing medical device and medical device producing apparatus)

The method for producing a medical device of the present invention includes a layer forming step and a hardening liquid delivering step, and as needed, further includes a removing step, a sintering step, a step of delivering at least any one of a cell and a growth factor, and other steps.

The medical device producing apparatus includes a layer forming unit and a hardening liquid delivering unit, and as needed, further includes a removing unit, a sintering unit, a unit configured to deliver at least any one of a cell and a growth factor, and other units.

The medical device is formed of a liquid (hereinafter may also be referred to as "slurry") containing ceramic particles having a diameter of less than 1 $\mu$m and an organic compound A, and a hardening liquid containing an organic compound B that exhibits a cross-linking reaction with the organic compound A. The porous portion and the dense portion can be controlled using process conditions that, for example, the number of discharging channels of a head is reduced for hardening the porous portion, and all discharging channels are used for hardening the dense portion. In existing modeling methods using laser or electron beams, there is a need for conveying powders. When ceramic particles such as zirconia particles that need sintering are provided with a small particle diameter in order to be ensured sinterability, fluidity of the ceramic particles is severely degraded, so the particles may not be able to be conveyed. In existing methods using laser or electron beams, heat may diffuse and melt nearby particles to degrade accuracy. However, in modeling methods using inkjet systems, accurate discharging on a picoliter order and hence finer modeling are available. Hence, it is possible to form the porous portion and the dense portion to have different textures needed, by optimizing the process conditions and more specifically the discharging conditions.

<Layer forming step and layer forming unit>

The layer forming step is a step of forming a liquid layer using a liquid containing ceramic particles having a volume average particle diameter of less than 1 $\mu$m and an organic compound A.

The layer forming unit is a unit configured to form a liquid layer using a liquid containing ceramic particles having a volume average particle diameter of less than 1 $\mu$m and an organic compound A.

The layer forming step can be performed favorably by the layer forming unit.

-Liquid-

The liquid contains ceramic particles and an organic compound A and further contains other components as needed.

--Ceramic particles--

The ceramic particles are not particularly limited and may be appropriately selected depending on the intended purpose. Examples of the ceramic particles include zirconia particles, alumina particles, silica particles, and lithium disilicate particles. One of these kinds of ceramic particles may be used alone or two or more of these kinds of ceramic particles may be used in combination. Among these kinds of ceramic particles, zirconia particles are preferable. When zirconia particles are used as the ceramic particles, the ceramic particles may contain yttria or ceria as a stabilizer.

The volume average particle diameter of the ceramic particles is less than 1 $\mu$m in the liquid. When the volume average particle diameter of the ceramic particles is less than 1 $\mu$m, a green sheet or a green body of the ceramic particles can be prevented from being low in density, can be sintered favorably, and can be improved in mechanical strength. The volume average particle diameter of the ceramic particles can be measured with a known particle diameter measuring instrument that is not particularly limited and may be appropriately selected depending on the intended purpose. For example, the volume average particle diameter of the ceramic particles can be measured with MULTISIZER III (available from Beckman Coulter Inc.) or FPIA-3000 (available from Sysmex Corporation) according to a known method. The green sheet or the green body is a sheet or a molded body obtained by subjecting a compound, which is a kneaded product of a slurry and a binder, to injection molding.

The zirconia particles have an extremely high melting point. Therefore, the zirconia particles may not be able to be sintered without a small volume average particle diameter. An ideal volume average particle diameter of the zirconia particles is on the order of some tens of nanometers. When the volume average particle diameter of the zirconia particles is 1 $\mu$m or greater, large gaps may remain between the particles to make the particles difficult to sinter. For performing typical lamination modeling, it is preferable to convey materials including the zirconia particles from a supplying tank to a printing tank. When the size of the particles constituting the materials is small, a strong interparticle force tends to act to significantly degrade fluidity of the particles. Hence, in order to maintain sinterability of the zirconia particles while improving fluidity of the zirconia particles, it is preferable to make the zirconia particles handleable in a slurry state maintained at a volume average particle diameter of an order of some hundreds of nanometers or less.

The content of the ceramic particles is preferably 20 parts by mass or greater but 70 parts by mass or less relative to 100 parts by mass of the liquid (slurry). When the content of the ceramic particles is 20 parts by mass or greater, it is possible to relatively reduce the amount of solvents that volatilize and provide a green sheet or a green body with a high density. When the content of the ceramic particles is 70 parts by mass or less, it is possible to improve fluidity of the ceramic particles as a slurry and convey the slurry preferably with, for example, a doctor blade.

--Organic compound A--

The organic compound A is not particularly limited and may be appropriately selected depending on the intended purpose. Examples of the organic compound A include water-soluble resins. Water solubility of the water-soluble resins means a solubility of 10% by mass or greater in water at room temperature (25 degrees C).

The organic compound A preferably contains an acidic functional group reactive with a basic functional group.

Examples of the acidic functional group include a carboxyl group and a hydroxyl group.

Examples of the organic compound A containing the acidic functional group include modified polyvinyl alcohols (PVA) and polyacrylic acids (PAA). One of these organic compounds A may be used alone or two or more of these organic compounds A may be used in combination. Among these organic compounds A, polyacrylic acids (PAA) are preferable because polyacrylic acids (PAA) have a high reactivity with a basic functional group.

The weight average molecular weight (Mw) of the organic compound A is preferably 400,000 or greater, more preferably 400,000 or greater but 1,000,000 or less, and particularly preferably 600,000 or greater but 800,000 or less. When the weight average molecular weight (Mw) of the organic compound A is 400,000 or greater, the organic compound A can easily build a cross-linked structure with the organic compound B contained in the hardening liquid. This makes a hardening time taken to harden the medical device appropriate. Meanwhile, when the weight average molecular weight (Mw) of the organic compound A is 1,000,000 or less, there is an advantage that the slurry has an appropriate viscosity, so the ceramic particles do not become uneven in the obtained slurry. For example, the weight average molecular weight (Mw) of the organic compound A can be calculated based on a molecular weight distribution of the isolated organic compound A obtained by gel permeation chromatography (GPC).

The content of the organic compound A is preferably 5 parts by mass or greater but 30 parts by mass or less relative to 100 parts by mass of the ceramic particles. When the content of the organic compound A is 5 parts by mass or greater, a sufficient binding effect can be obtained to make the dispersion state of the ceramic particles in the slurry good and improve dispersion stability. Meanwhile, when the content of the organic compound A is 30 parts by mass or less, the slurry can be suppressed in viscosity and can be conveyed favorably with, for example, a doctor blade. The content of the organic compound A can be measured with a known thermal analyzer that is not particularly limited and may be appropriately selected depending on the intended purpose. For example, the content of the organic compound A can be measured with DSC-200 (available from Seiko Instruments Inc.) according to a known method.

--Other components--

The other components are not particularly limited and may be appropriately selected depending on the intended purpose. Examples of the other components include a solvent, a dispersant, a plasticizer, and a sintering aid.

---Solvent---

The solvent is not particularly limited and may be appropriately selected depending on the intended purpose so long as the solvent can dissolve the organic compound A. Examples of the solvent include polar solvents such as water, methanol, ethanol, and toluene (boiling point: 110.6 degrees C). One of these solvents may be used alone or two or more of these solvents may be used in combination. Among these solvents, organic solvents having a low boiling point are preferable and organic solvents having a boiling point of 80 degrees C or lower are more preferable in terms of improving productivity in forming a green sheet or a green body.

Examples of the organic solvents having a boiling point of 80 degrees C or lower include ethanol (boiling point: 78.37 degrees C), methanol (boiling point: 64.7 degrees C), ethyl acetate (boiling point: 77.1 degrees C), acetone (boiling point: 56 degrees C), and methylene chloride (boiling point: 39.6 degrees C).

It is preferable that the liquid contain the dispersant, because the dispersant improves dispersibility of the ceramic particles, can suppress sedimentation of the ceramic particles during a still state, and makes it easier for the inorganic particles to be present continuously during formation of a green sheet or a green body. It is preferable that the liquid contain the plasticizer, because the plasticizer makes it harder for a green sheet or green body precursor formed of the liquid to be cracked when dried. It is preferable that the liquid contain the sintering aid, because the sintering aid makes a laminated object to be obtained sinterable at a lower temperature during sintering.

The plasticizer is not particularly limited and may be appropriately selected depending on the intended purpose. Examples of the plasticizer include benzyl butyl phthalate.

[Formation of liquid layer]

A method for placing the liquid on a support is not particularly limited and may be appropriately selected depending on the intended purpose. Preferable examples of a method for placing the liquid (slurry material) as, for example, a thin layer include: a method using a known counter rotation mechanism (counter roller), the method being used in a selective laser sintering method described in Japanese Patent No. 3607300; a method of spreading the slurry material into a thin layer using such a member as a brush, a roller, or a blade; a method of spreading a layer of the slurry material into a thin layer by pressing the surface of the layer with a press member; and a method using a known powder lamination modeling apparatus.

For example, the following procedure may be performed in order to place the slurry material on the support using, for example, the counter rotation mechanism (counter roller), the brush, roller, or blade, or the press member. That is, for example, the support may be disposed within an outer frame (may also be referred to as "mold", "hollow cylinder", and "tubular structure") in a manner that the support can be lifted up and down while sliding on the internal wall of the outer frame. The slurry material may be placed on this support using, for example, the counter rotation mechanism (counter roller), the brush, roller, or blade, or the press member. When the support used is a member that can be lifted up and down within the outer frame, the support may be disposed at a position slightly below the upper end opening of the outer frame, i.e., at a position below the upper end opening by what corresponds to a thickness of the liquid layer (slurry material layer), and then the slurry material may be placed on the support. In this way, the slurry material can be placed on the support as a thin layer.

When the hardening liquid is caused to act on the slurry material placed as a thin layer in the manner described above, the thin layer is hardened. When the slurry material is placed as a thin layer in the same manner as described above on the hardened product of the thin layer just obtained and the hardening liquid is caused to act on the slurry material (layer) placed as a thin layer, hardening occurs. This hardening occurs not only in the slurry material (layer) placed as a thin layer, but also between the slurry material (layer) and the underlying hardened product of the thin layer obtained by the previous hardening. As a result, a hardened product (medical device) having a thickness corresponding to about two layers of the slurry material (layer) placed as a thin layer is obtained.

Alternatively, an automatic, quick manner using the known powder lamination modeling apparatus may be employed to place the liquid as a thin layer on the support. Typically, the powder lamination modeling apparatus includes a recoater configured to laminate a layer of the slurry material, a movable supplying tank configured to supply the slurry material onto the support, and a movable forming tank in which the slurry material is placed as a thin layer and laminated. In the powder lamination modeling apparatus, it is possible to constantly dispose the surface of the supplying tank slightly above the surface of the forming tank by lifting up the supplying tank, by lifting down the forming tank, or by both, it is possible to place the slurry material as a thin film by actuating the recoater from the supplying tank side, and it is possible to laminate thin layers of the slurry material by repeatedly moving the recoater. This powder lamination modeling apparatus as is may be used for slurry lamination, or the recoater member may be changed to a doctor blade for sheet modeling.

The average thickness of the liquid layer is not particularly limited and may be appropriately selected depending on the intended purpose. For example, the average thickness of the liquid layer per layer is preferably 3 $\mu$m or greater but 200 $\mu$m or less and more preferably 10 $\mu$m or greater but 100 $\mu$m or less. When the average thickness of the liquid layer is 3 $\mu$m or greater, the time taken until the medical device is obtained is adequate, and the medical device will not have problems such as shape collapse during treatment or handling such as sintering. Meanwhile, when the average thickness of the liquid layer is 200 $\mu$m or less, the medical device has a sufficient dimensional accuracy. The average thickness of the liquid layer can be measured according to a known method.

-Support-

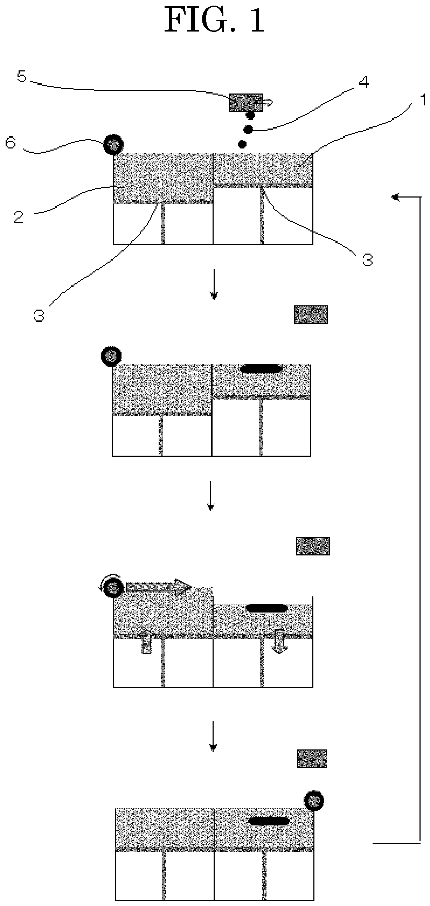

The support (liquid material layer holding unit) is not particularly limited and may be appropriately selected depending on the intended purpose so long as the liquid can be placed on the support. Examples of the support include a table having a placing surface on which the liquid is placed, and a base plate of an apparatus illustrated in FIG. 1 of Japanese Unexamined Patent Application Publication No. 2000-328106. The surface of the support, i.e., the placing surface on which the liquid is placed, may be, for example, a smooth surface, a coarse surface, a flat surface, or a curved surface.

<Hardening liquid delivering step and hardening liquid delivering unit>

The hardening liquid delivering step is a step of delivering a hardening liquid containing an organic compound B that exhibits a cross-linking reaction with the organic compound A to a predetermined region of the liquid layer.

The hardening liquid delivering unit is a unit configured to deliver a hardening liquid containing an organic compound B that exhibits a cross-linking reaction with the organic compound A to a predetermined region of the liquid layer.

The hardening liquid delivering step can be performed favorably by the hardening liquid delivering unit.

-Hardening liquid-

The hardening liquid contains an organic compound B that exhibits reactivity with the organic compound A, and further contains other components as needed.

--Organic compound B--

The organic compound B is not particularly limited and may be appropriately selected depending on the intended purpose so long as the organic compound B exhibits a cross-linking reaction with the organic compound A. Examples of the organic compound B includes water-soluble resins. Water solubility of the water-soluble resins means a solubility of 10% by mass or greater in water at room temperature (25 degrees C).

The organic compound B preferably contains a basic functional group reactive with an acidic functional group.

Examples of the basic functional group include an amino group.

Examples of the organic compound B containing the amino group include polyethylenimine and polyvinylpyrrolidone. One of these organic compounds B may be used alone or two or more of these organic compounds B may be used in combination. Among these organic compounds B, polyethylenimine having a high cation density is preferable in terms of reactivity with an acidic functional group.

The weight average molecular weight (Mw) of the organic compound B is preferably 1,800 or greater, more preferably 1,800 or greater but 70,000 or less, and particularly preferably 3,000 or greater but 20,000 or less. When the weight average molecular weight (Mw) of the organic compound B is 1,800 or greater, the organic compound B can easily build a cross-linked structure with the organic compound A contained in the liquid and having an acidic functional group. This makes a hardening time taken to harden the medical device appropriate. Meanwhile, when the weight average molecular weight (Mw) of the organic compound B is 70,000 or less, there is an advantage that the hardening liquid has an appropriate viscosity and can be discharged stably. For example, the weight average molecular weight (Mw) of the organic compound B can be measured by gel permeation chromatography (GPC).

The content of the organic compound B is preferably 3 parts by mass or greater but 20 parts by mass or less relative to 100 parts by mass or the hardening liquid. When the content of the organic compound B is 3 parts by mass or greater, the organic compound B can sufficiently build a cross-linked structure with the organic compound A contained in the liquid and can improve the strength of a green sheet or a green body to be obtained. Meanwhile, when the content of the organic compound B is 20 parts by mass or less, the hardening liquid can be suppressed in viscosity and can be improved in discharging stability.

The content of the organic compound B can be measured with a known thermal analyzer that is not particularly limited and may be appropriately selected depending on the intended purpose. For example, the content of the organic compound B can be measured with DSC-200 (available from Seiko Instruments Inc.) according to a known method.

The hardening liquid can be used favorably for easy, efficient production of various medical devices.

--Other components--

The other components are not particularly limited and may be appropriately selected depending on the intended purpose. Examples of the other components include an aqueous medium, a surfactant, a preservative, an antiseptic, a stabilizer, and a pH regulator.

---Aqueous medium---

Examples of the aqueous medium include water, alcohols such as methanol and ethanol, ethers, and ketones.

One of these aqueous media may be used alone or two or more of these aqueous media may be used in combination. Among these aqueous media, water is preferable. The aqueous medium may be water that contains other components than water, such as the alcohols in a small amount.

Examples of the water include pure water such as ion-exchanged water, ultrafiltrated water, reverse osmotic water, and distilled water, and ultrapure water.

The method for delivering the hardening liquid to the liquid layer is not particularly limited and may be appropriately selected depending on the intended purpose. Examples of the method include liquid discharging units employed in, for example, a dispenser method, a spray method, and an inkjet method. In the present invention, it is preferable to use a liquid discharging unit (configured to discharge liquid droplets from a plurality of nozzles using a vibration element such as a piezoelectric actuator) employed in the inkjet method, because this liquid discharging unit enables accurate, efficient formation of a complex three-dimensional shape.

<Removing step and removing unit>

The removing step is a step of immersing the medical device formed by sequentially repeating the layer forming step and the hardening liquid delivering step in a solvent to remove any unreacted slurry material.

The removing unit is a unit configured to immerse the medical device formed by sequentially and repeatedly activating the layer forming unit and the hardening liquid delivering unit in a solvent to remove any unreacted slurry material.

Examples of the solvent include a sodium hydroxide aqueous solution.

<Sintering step and sintering unit>

The sintering step is a step of sintering the medical device (laminated object) formed by sequentially repeating the layer forming step and the hardening liquid delivering step. The sintering step is performed by the sintering unit. By performing the sintering step, it is possible to form an integrated compact (sintered body) of the hardened product.

Examples of the sintering unit include a known sintering furnace.

<Delivering step and delivering unit for delivering at least any one of cell and growth factor>

The step of delivering at least any one of a cell and a growth factor is a delivering step of discharging at least any one of a cell and a growth factor from an inkjet nozzle to deliver the at least any one of the cell and the growth factor to a predetermined region.

The unit configured to deliver at least any one of a cell and a growth factor is a delivering unit configured to discharge at least any one of a cell and a growth factor from an inkjet nozzle to deliver the at least any one of the cell and the growth factor to a predetermined region.

The step of delivering at least any one of a cell and a growth factor can be performed favorably by the unit configured to deliver at least any one of a cell and a growth factor.

By performing the delivering step, it is possible to produce a medical device in which the sintered body and the cell or the growth factor are integrated with each other.

The predetermined region refers to a predetermined region in the compact (sintered body).

The predetermined region in the compact (sintered body) is preferably the porous portion of the medical device.

--Cell and growth factor--

As the cell, the same cell as in the medical device of the present invention may be used.

The cell can be delivered in the form of a cell dispersion liquid obtained by dispersing the cell in an aqueous medium.

The cell dispersion liquid contains the cell and the aqueous medium, and further contains other components as needed. The cell dispersion liquid can impart a function in a favorable manner by being delivered to the surface of the medical device.

-Aqueous medium-

The aqueous medium is not particularly limited and may be appropriately selected depending on the intended purpose. Examples of the aqueous medium includes a simulated body fluid, a TE buffer liquid, and a culture medium. One of these aqueous media may be used alone or two or more of these aqueous media may be used in combination.

-Other components-

The other components are not particularly limited and may be appropriately selected depending on the intended purpose. Examples of the other components include a surfactant, a dispersant, an antibiotic, a chelate agent, and a pH regulator.

As the growth factor, the same growth factor as in the medical device of the present invention may be used.

The growth factor can be delivered in the form of a growth factor-containing liquid obtained by adding the growth factor in an aqueous medium.

The growth factor-containing liquid contains the growth factor and the aqueous medium, and further contains other components as needed.

-Aqueous medium-

The aqueous medium may be the same as the aqueous medium used in the cell dispersion liquid.

-Other components-

The other components may be the same as the other components used in the cell dispersion liquid.

<Other steps and other units>

Examples of the other steps include a surface protecting step and a painting step. Examples of the other units include a surface protecting unit and a painting unit.

-Surface protecting step and surface protecting unit-

The surface protecting step is a step of forming a protective layer on the medical device formed in the hardening liquid delivering step or the sintering step. By performing the surface protecting step, it is possible to provide the surface of the medical device with, for example, durability that enables the medical device to be subjected to use, etc. as is.

Examples of the protective layer include a water-resistant layer, a weather-resistant layer, a light-resistant layer, a heat insulating layer, and a gloss layer.

Examples of the surface protecting unit include known surface protecting treatment machines such as a spray machine and a coating machine.

-Painting step and painting unit-

The painting step is a step of painting the medical device. By performing the painting step, it is possible to color the medical device in a desired color. Examples of the painting unit include known painting machines such as painting machines using, for example, a spray, a roller, and a brush.

FIG. 1 illustrates an example of a medical device producing apparatus. The medical device producing apparatus illustrated in FIG. 1 includes a forming-side slurry storing tank 1 and a supplying-side slurry storing tank 2. These slurry storing tanks each include a stage 3 movable up and down. A layer of the slurry material is formed on the stage.

There is provided an inkjet head 5 above the forming-side slurry storing tank 1. The inkjet head 5 is configured to discharge a hardening liquid 4 to the liquid (slurry material) in the slurry storing tank. There is also provided a leveling mechanism 6 (hereinafter may also be referred to as recoater) configured to supply the slurry material from the supplying-side slurry storing tank 2 to the forming-side slurry storing tank 1 and level the surface of the slurry material layer in the forming-side slurry storing tank 1.

The hardening liquid 4 is dropped from the inkjet head 5 onto the slurry material in the forming-side slurry storing tank 1. The position to which the hardening liquid 4 is dropped is determined based on two-dimensional image data (slice data) representing a plurality of planer layers into which a three-dimensional shape finally desired is sliced.

When printing on one layer is completed, the stage 3 of the supplying-side slurry storing tank 2 is lifted up, and the stage 3 of the forming-side slurry storing tank 1 is lifted down, which produces a height difference. An amount of the slurry material corresponding to the height difference is moved to the forming-side slurry storing tank 1 by the leveling mechanism 6.

In this way, one new layer of the slurry material is formed on the surface of the slurry material layer on which printing is performed previously. The thickness of this one layer of the slurry material is approximately greater than or equal to some tens of micrometers but less than or equal to 100 micrometers. Printing is performed on this newly formed slurry material layer based on the slice data of the second layer. This series of process is repeated to obtain a medical device. The medical device is heated and dried by an unillustrated heating unit to obtain the final object.

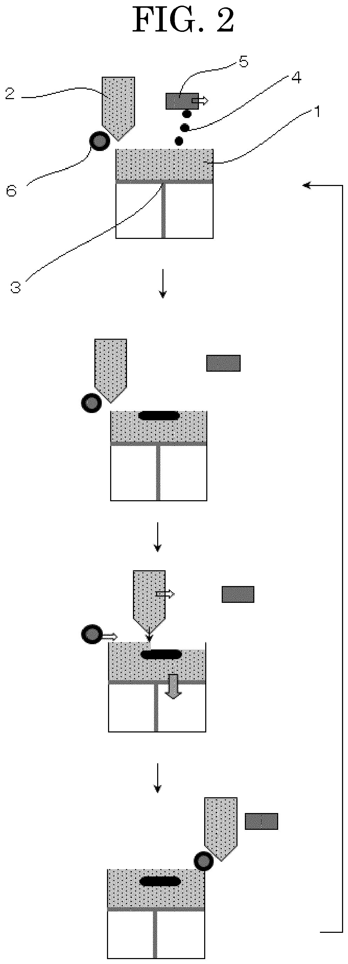

FIG. 2 illustrates another example of a slurry lamination modeling apparatus. The method for producing a medical device illustrated in FIG. 2 is the same as in FIG. 1 in principle, but is different in the mechanism of supplying the liquid (slurry material). That is, the supplying-side slurry storing tank 2 is disposed above the forming-side slurry storing tank 1. When printing on the first layer is completed, the stage 3 of the forming-side slurry storing tank 1 lifts down by a predetermined distance, and the supplying-side slurry storing tank 2 moves while dropping the slurry material in a predetermined amount into the forming-side slurry storing tank 1 to form a new slurry material layer. Subsequently, the leveling mechanism 6 compresses the liquid (slurry material) layer to increase the bulk density and level off the slurry material layer to a uniform height.

The medical device producing apparatus having the configuration illustrated in FIG. 2 can be made smaller in size than the configuration of FIG. 1 in which two slurry storing tanks are arranged horizontally.

The method for producing a medical device of the present invention and the medical device producing apparatus can produce a medical device having a complex three-dimensional shape easily and efficiently using the liquid, the hardening liquid, etc. of the present invention. The medical device obtained in this way has a good adhesiveness with a biological (soft) tissue, a desired surface profile, and an excellent dimensional accuracy, and can reproduce, for example, minute asperity and curved surfaces. Therefore, the medical device has an excellent aesthetic appearance and a high quality, and can be used favorably for various purposes.

Cytotoxicity assay for the medical device is not particularly limited, and a known in-vitro assay may be appropriately selected depending on the intended purpose. Examples of the cytotoxicity assay include: (i) a MTT assay for colorimetric activity, for measuring an activity of a mitochondrial reductase using tetrazolium salt 3-(4,5-dimethylthiazol-2-yl)-2,5-diphenyltetrazolium bromide; (ii) similar assays using any other tetrazolium salt and a formazan dye, such as XTT and WST assays; (iii) a trypan blue (TB) assay; (iv) a sulforhodamine B (SRB) assay; and (v) a clonogenic assay.

Furthermore, any methods known to the persons skilled in the art for measuring necrosis and apoptosis levels of cells may be used to determine whether a cationic lipid or a drug has a cytotoxic activity.

Examples of the method for measuring apoptosis include, but are not limited to, a TUNEL assay, caspase activity measurement, DNA fragmentation, poly(ADP-ribose)polymerase (PARP) activation, mitochondrial cytochrome c release, apoptosis-inducing factor (AIF) migration, and Annexin-V staining.

EXAMPLES

The present invention will be described below by way of Examples. The present invention should not be construed as being limited to the Examples.

Examples and Comparative Examples below present examples in which laminated objects were produced using three-dimensional object modeling (lamination modeling) and without using a mold. However, these examples are non-limiting examples.

The voidage of a medical device was calculated according to a formula below.

$$\text{Voidage (\%)} = [(\text{true density} - \text{tap density})/\text{true density}] \times 100$$

The true density and the tap density in the formula were measured with a powder characteristics tester (instrument name: POWDER TESTER (registered trademark) PT-X, available from Hosokawa Micron Group).

(Liquid (slurry material) preparation example 1)

<Synthesis of ceramic particles 1>

A 18% by mass yttrium chloride aqueous solution was mixed with a 20% by mass zirconium oxychloride aqueous solution such that an equivalent yttria-zirconia ratio by mole (yttria:zirconia) would be 2.8:97.2. Sodium chloride was added and dissolved in the resultant in an amount of 0.5% by mass of the total amount of zirconium oxychloride.

Subsequently, aluminum chloride was added and dissolved in the obtained aqueous solution in an amount that would provide alumina in an amount of 0.4% by mass of the total amount of zirconia. The resultant aqueous solution was subjected to spray drying in air at a temperature of 200 degrees C, to obtain dry powder. The obtained dry powder was fired in air at a temperature of 1,000 degrees C, to synthesize calcined powder. The obtained calcined powder had a monoclinic phase ratio of 8.2%. The calcined powder was pulverized with a wet attritor, to obtain a 30% by mass slurry. Subsequently, the obtained slurry was repeatedly subjected to dilution and filtration concentration through a membrane filter having a mesh size of 0.5 μm, to be repeatedly washed until an electrical conductivity of the filtrate became 20 μS

or lower, to synthesize ceramic particles 1 (zirconia particles).

<Preparation of liquid (slurry material) 1>

The ceramic particles 1 (zirconia particles) (130.0 parts by mass), polyacrylic acid (PAA, available from Nippon Shokubai Co., Ltd., AS-58) (5.0 parts by mass), benzyl butyl phthalate as a plasticizer (available from Wako Pure Chemical Industries, Ltd.) (10.0 parts by mass), a ceramic dispersant (MARIARIM, available from NOF Corporation, AKM-0531) (1.5 parts by mass), ethanol (60 parts by mass) were mixed and subjected to dispersion treatment for 3 hours using a bead mill using zirconia beads having a diameter of 3 mm, to obtain a liquid (slurry material) 1.

The volume average particle diameter of the ceramic particles contained in the obtained liquid (slurry material) 1 was measured in the manner described below.

-Volume average particle diameter of ceramic particles-

The volume average particle diameter of the ceramic particles contained in the liquid (slurry material) 1 was measured with instrument name: LA-920 (available from Horiba Ltd.). During the measurement with LA-920, an analysis was performed using an application (Ver. 3.32) (available from Horiba Ltd.) dedicated for LA-920. Specifically, after optical axis adjustment with chloroform, background measurement was performed. Subsequently, circulation was started to drop the liquid (slurry material). After it was confirmed that the transmittance was stabilized, ultrasonic irradiation was performed under conditions described below. After irradiation, the volume average particle diameter was measured under a condition that the transmittance value was in the range of 70% or higher but 95% or lower. In terms of repeatability of the measurement of volume average particle diameter, the measurement was performed under a condition that the transmittance value of LA-920 was 70% or higher but 95% or lower. When the transmittance came out of the range as a result of the ultrasonic irradiation, the measurement was performed again. The amount of the liquid (slurry material) dropped was adjusted in a manner to obtain a transmittance value in the range. The measurement and analysis conditions were set as follows.

[Measurement and analysis conditions]

- Number of times data was taken: 15 times
- Relative refractive index: 1.20
- Circulation: 5
- Ultrasonic intensity: 7

(Liquid (slurry material) preparation examples 2 to 5)

<Preparation of liquids (slurry materials) 2 to 5>

Liquids (slurry materials) 2 to 5 were obtained in the same manner as in Liquid (slurry material) preparation example 1, except that the composition used in Liquid (slurry material) preparation example 1 was changed to the compositions presented in Table 1 below. The volume average particle diameters of the ceramic particles were measured in the same manner as in Liquid (slurry material) preparation example 1.

The compositions of the liquids (slurry materials) 1 to 5 and the volume average particle diameters of the ceramic particles are presented in table 1 below.

Table 1

| | | Liquid | | | | |
|---|---|---|---|---|---|---|
| | | 1 | 2 | 3 | 4 | 5 |
| Ceramic particles | Zirconia particles | 130.0 | 130.0 | - | - | - |
| | Lithium disilicate particles | - | - | 130.0 | - | 130.0 |
| | Alumina particles | - | - | - | 130.0 | - |

(continued)

| | | Liquid | | | | |
|---|---|---|---|---|---|---|
| | | 1 | 2 | 3 | 4 | 5 |
| Organic compound A | Polyacrylic acid | 5.0 | - | 5.0 | 5.0 | 5.0 |
| | Modified polyvinyl alcohol | - | 5.0 | - | - | - |
| Plasticizer | Benzyl butyl phthalate | 10.0 | 10.0 | 10.0 | 10.0 | 10.0 |
| Dispersant | Ceramic dispersant | 1.5 | 1.5 | 1.5 | 1.5 | 1.5 |
| Solvent | Ethanol | 60.0 | 60.0 | 60.0 | 60.0 | 60.0 |
| Volume average particle diameter ($\mu$m) of ceramic particles | | 0.2 | 0.2 | 0.2 | 0.2 | 1.0 |

The product names and supplier names of the components presented in Table 1 are as follows.

- Lithium disilicate particles: synthesized product
- Alumina particles: available from Nippon Light Metal Company, Ltd., product name: MM-22
- Polyacrylic acid: available from Nippon Shokubai Co., Ltd., product name: AS-58
- Modified polyvinyl alcohol: available from Japan Vam & Poval Co., Ltd., product name: AHF-17
- Benzyl butyl phthalate: available from Wako Pure Chemical Industries, Ltd.
- Ceramic dispersant: available from NOF Corporation, product name: MARIARIM (registered trademark) AKM-0531

(Hardening liquid preparation example 1)

<Preparation of hardening liquid 1>

Water (88.0 parts by mass), polyethylenimine (PEI, available from Nippon Shokubai Co., Ltd., SP-200) (12.0 parts by mass), and a surfactant TWEEN 20 (available from Tokyo Chemical Industry Co., Ltd.) (0.5 parts by mass) were subjected to dispersion treatment for 30 minutes using a homomixer, to obtain a hardening liquid 1.

(Hardening liquid preparation example 2)

<Preparation of hardening liquid 2>

A hardening liquid 2 was obtained in the same manner as in Hardening liquid preparation example 1, except that the composition used in Hardening liquid preparation example 1 was changed to the composition presented in Table 2 below.

The compositions of the hardening liquids 1 and 2 are presented in Table 2 below.

Table 2

| | | Hardening liquid | |
|---|---|---|---|
| | | 1 | 2 |
| Organic compound B | Polyethylenimine | 12.0 | - |
| | Polyvinylpyrrolidone | - | 12.0 |
| Surfactant | TWEEN 20 | 0.5 | 0.5 |
| Water | | 88.0 | 88.0 |

The product names and supplier names of the components presented in Table 2 are as follows.

- Polyethylenimine (PEI): available from Nippon Shokubai Co., Ltd., product name: SP-200, weight average molecular weight (Mw): 10,000
- Polyvinylpyrrolidone (PVP): available from Nippon Shokubai Co., Ltd., product name: K-30, weight average molecular weight (Mw): 10,000
- TWEEN 20: available from Tokyo Chemical Industry Co., Ltd.

(Example 1)

A medical device (laminated object) 1 was produced according to the procedures (1) to (3) below, using the obtained liquid (slurry material) 1 and the hardening liquid 1, and a shape printing pattern having a size of 70 mm in length and 12 mm in width.

(1) First, using a medical device producing apparatus as illustrated in FIG. 1, the slurry material 1 was moved from the supplying-side slurry storing tank to the forming-side slurry storing tank, to form a thin layer formed of the slurry material 1 having an average thickness of 100 $\mu$m on the support.

(2) Next, the hardening liquid 1 was delivered (discharged) from nozzles onto the surface of the formed thin layer of the slurry material 1 using an inkjet printer (available from Ricoh Company, Ltd., SG7100), to harden the slurry material 1. At the time, the process conditions were controlled such that the number of discharging channels from the head was reduced for hardening the porous portion, and all discharging channels were used for hardening the dense portion, to adjust the amount of the hardening liquid 1 to be delivered.

(3) Next, the operations of (1) and (2) were repeated until a predetermined total average thickness of 3 mm was obtained, and hardened thin layers of the slurry material 1 were sequentially laminated, to obtain a hardened product. The obtained hardened product was left to stand at normal temperature and dried for the solvents to volatilize, to produce a medical device. The obtained medical device was immersed in water to remove any unhardened slurry material components. As a result, the medical device did not undergo a shape collapse. The surface of the medical device had a porous portion (with a voidage of 5% or greater) and a dense portion (with a voidage of less than 5%), as a result of the control of the process conditions that the number of discharging channels from the head was reduced for hardening the porous portion and all discharging channels were used for hardening the dense portion.

(Examples 2 to 12 and Comparative Examples 1 to 3)

Medical devices were produced in the same manner as in Example 1, except that the combination of the liquid and the hardening liquid used in Example 1 was changed to the combinations presented in Table 3 below. The surfaces of the produced medical devices had porous portions (with a voidage of 5% or greater) and dense portions (with a voidage of less than 5%) as a result of the control of the process conditions.

<Dimensional accuracy>

Next, the obtained medical devices were visually observed to evaluate dimensional accuracy according to evaluation criteria described below. The results are presented in Table 3 below.

[Evaluation criteria]

A: The surface of the obtained medical device was smooth and beautiful, and had no warpage.
B: The surface of the obtained medical device had a slight distortion ad a slight warpage.
C: The surface of the obtained medical device had distortion and a severe warpage.

After dimensional accuracy of the medical device obtained in (3) described above was evaluated, the medical device was sintered in the manner (4) described below, to produce a sintered body of the medical device after sintered.
(4) A medical device using zirconia particles as the ceramic particles was sintered in a sintering furnace under an air atmosphere at 1,500 degrees C.
A medical device using lithium disilicate particles as the ceramic particles was sintered under an air atmosphere at 900 degrees C.
A medical device using alumina particles as the ceramic particles was sintered under an air atmosphere at 1,200 degrees C.
The sintered bodies of these medical devices were completely integrated structures and did not undergo breakage, etc. even when slammed on a hard floor.

The arithmetic average roughness (Ra) of the surfaces of the medical devices after sintered in (4) was measured according to JIS B 0601:2013.

<Preparation of cell dispersion liquid>

Frozen cells of a mouse calvaria-derived cell MC3T3-E1 cell line (available from DS Pharma Biomedical Co., Ltd., at a cell concentration of $1.0 \times 10^5$ cells/mL) (10 mL) and a 10% by mass aqueous medium of Alpha-MEM+FBS (available from DS Pharma Biomedical Co., Ltd.) (10 mL) were mixed, to obtain a cell dispersion liquid.

<Preparation of growth factor-containing liquid>

Fibronectin, which was a growth factor (cell adhesion factor, product name: FIBRONECTIN SOLUTION, derived from human plasma, available from Wako Pure Chemical Industries, Ltd.) (10 mL) was diluted 10-fold with 20 mmol/L Tris-HCl, 450 mmol/L NaCl, and a 12% by mass glycerol aqueous solution, to obtain a growth factor-containing liquid.

Next, as presented in Table 3 below, the cell dispersion liquid or the growth factor-containing liquid was delivered (discharged) from nozzles onto the surfaces of the medical devices after sintered obtained in (4), using an inkjet printer (available from Ricoh Company, Ltd., SG7100), such that the liquid was attached on the surfaces in an amount of 30 $\mu$g/cm$^2$, to immobilize the cell or the growth factor on the surfaces of the medical devices.

<Cell adhesiveness of medical devices>

About $3 \times 10^4$ V79 cells (Chinese hamster lung-derived fibroblast) were seeded in a cell culture liquid (a 5% by mass fetal bovine serum-added MEM culture medium) contained in a well and left to stand still at 37 degrees C for 4 hours. Subsequently, the medical device 1 was put in the well. In this state, the cells were cultured for 2 weeks with the culture medium replaced every other day. Subsequently, the number of cells on the porous portion and the dense portion of the medical device was measured with an automatic cell counter (product name: COUNTESS AUTOMATED CELL COUNTER, available from Invitrogen Corporation). Subsequently, based on the obtained number of cells, the cell proliferation ratio (%) was calculated according to a formula below. Cell "adhesiveness" was evaluated according to evaluation criteria described below based on the obtained cell proliferation ratio (%).

Cell proliferation ratio (%) = (number of cells on medical device after 2-week culture)/(number of cells on medical device before culture)$\times$100

[Evaluation criteria for porous portion]

A: The cell proliferation ratio was 300% or greater.
B: The cell proliferation ratio was 100% or greater but less than 300%.
C: The cell proliferation ratio was less than 100%.

[Evaluation criteria for dense portion]

A: The cell proliferation ratio was less than 100%.
B: The cell proliferation ratio was 100% or greater but less than 300%.
C: The cell proliferation ratio was 300% or greater.

<Tissue adhesiveness of porous portion>

Only the porous portion of the medical device was implanted in a gingival tissue of a 12-week-old ICR male mouse (purchased from Sankyo Labo Service Corporation, Inc.), and the status of the porous portion was visually observed to evaluate "tissue adhesiveness" according to evaluation criteria described below.

[Evaluation criteria]

A: The porous portion of the medical device adhered to the surrounding tissue within 30 days, and no inflammation was caused in the space between the gingival tissue and the porous portion.

B: The porous portion of the medical device adhered to the surrounding tissue past 30 days but within 90 days, and no inflammation was caused in the space between the gingival tissue and the porous portion.

C: The porous portion of the medical device did not adhere to the surrounding tissue even past 90 days, and inflammation was caused in the space between the gingival tissue and the porous portion.

<Tissue adhesiveness of dense portion>

The back of a 12-week-old ICR male mouse (purchased from Sankyo Labo Service Corporation, Inc.) was cut open and subcutaneously implanted with only the dense portion of the medical device, and then the cut portion was sutured. "Tissue adhesiveness" was evaluated according to evaluation criteria described below.

[Evaluation criteria]

A: The obtained medical device almost had not adhered to the surrounding tissue even when 90 days passed, and was able to be removed easily by reopening.

B: The obtained medical device had adhered to the surrounding tissue when 90 days passed but almost had not adhered to the surrounding tissue when 30 day passed, and was able to be removed easily by reopening.

C: The obtained medical device had strongly adhered to the surrounding tissue when 30 days passed, and was not able to be removed without resection of the surrounding tissue with a scalpel.

Table 3

| | | Liquid | Hardening liquid | Cell | Growth factor | Arithmetic average roughness (Ra) ($\mu$m) | | Evaluation result | | | | Dimensional accuracy |
|---|---|---|---|---|---|---|---|---|---|---|---|---|
| | | | | | | | | Cell adhesiveness | | Tissue adhesiveness | | |
| | | | | | | Porous portion | Dense portion | Porous portion | Dense portion | Porous portion | Dense portion | |
| Ex. | 1 | 1 | 1 | - | - | 4.9 | 1.1 | A | A | B | A | A |
| | 2 | 2 | 1 | - | - | 5.2 | 1.3 | A | A | B | A | A |
| | 3 | 3 | 1 | - | - | 4.8 | 1.2 | A | A | B | A | A |
| | 4 | 4 | 1 | - | - | 5.1 | 1.1 | A | A | B | A | A |
| | 5 | 5 | 1 | - | - | 11.4 | 1.6 | A | A | B | A | A |
| | 6 | 1 | 2 | - | - | 5.6 | 1.3 | A | A | B | A | A |
| | 7 | 1 | 1 | MC3T3-E1 | - | 4.9 | 1.1 | A | A | A | A | A |
| | 8 | 1 | 1 | - | Fibronectin | 4.9 | 1.1 | A | A | A | A | A |
| | 9 | 1 | 1 | MC3T3-E1 | - | 4.9 | 1.1 | A | A | A | A | A |
| | 10 | 1 | 1 | - | - | 20.0 | 1.4 | B | A | B | A | B |
| | 11 | 1 | 1 | - | - | 2.0 | 0.8 | B | A | B | A | A |
| | 12 | 1 | 1 | - | - | 7.9 | 1.9 | A | A | B | B | A |
| Comp. Ex. | 1 | 1 | 1 | - | - | 21.0 | 1.4 | C | A | A | A | C |
| | 2 | 1 | 1 | - | - | 1.9 | 1.2 | C | A | C | A | A |
| | 3 | 1 | 1 | - | - | 4.8 | 2.0 | A | B | A | C | A |

**Claims**

1. A medical device comprising:

   a porous portion; and
   a dense portion,
   wherein an arithmetic average roughness of a surface of the porous portion is 2.0 $\mu$m or greater but 10 $\mu$m or less,
   wherein an arithmetic average roughness of a surface of the dense portion is less than 2.0 $\mu$m,
   wherein the porous portion has a voidage of 5% or greater,
   wherein the dense portion has a voidage of less than 5%,
   wherein the porous portion is a surface portion to contact the soft tissue,
   wherein the dense portion is a portion not to contact the soft tissue,
   wherein the porous portion and the dense portion comprise sintered ceramic particles, and
   wherein the medical device is an artificial product intended for compensating for defective portions of hard tissues.

2. The medical device according to claim 1,
   wherein the arithmetic average roughness of the surface of the dense portion is less than 1.0 $\mu$m.

3. The medical device according to claim 1 or claim 2,
   wherein the ceramic particles comprises at least one selected from the group consisting of zirconia, alumina, and lithium disilicate.

4. The medical device according to any one of claims 1 to 3,
   wherein the porous portion comprises at least any one of a cell and a growth factor.

5. The medical device according to claim 4,
   wherein the cell comprises at least one selected from the group consisting of a gingival fibroblast, a gingival epithelial progenitor cell, an osteoblast, and an osteoclast.

6. The medical device according to claim 4 or 5,
   wherein the growth factor comprises at least any one of an osteogenic factor and a cell adhesion factor.

7. The medical device according to any one of claims 1 to 6,
   wherein the medical device comprises a dental prosthesis.

8. The medical device according to claim 7,
   wherein the dental prosthesis comprises an artificial tooth.

9. A method for producing a medical device, the method comprising:

   forming a liquid layer using a liquid that comprises ceramic particles having a volume average particle diameter of less than 1 $\mu$m and an organic compound A;
   optimizing the discharge conditions to form the porous portion and the dense portion; and
   delivering a hardening liquid that comprises an organic compound B that exhibits a cross-linking reaction with the organic compound A to a predetermined region of the liquid layer, wherein the method repeats the forming and the delivering a plurality of times to produce a hardened product and sintering the hardened product to produce the medical device according to any one of claims 1 to 8.

10. The method for producing a medical device according to claim 9, the method further comprising
    discharging at least any one of a cell and a growth factor from an inkjet nozzle to deliver the at least any one of the cell and the growth factor to a predetermined region.

**Patentansprüche**

1. Medizinische Vorrichtung umfassend:

   einen porösen Teil; und

einen dichten Teil,

wobei eine arithmetisch gemittelte Rauheit einer Oberfläche des porösen Teils 2,0 $\mu$m oder mehr, aber 10 $\mu$m oder weniger beträgt,

wobei eine arithmetisch gemittelte Rauheit einer Oberfläche des dichten Teils weniger als 2,0 $\mu$m beträgt,

wobei der poröse Teil einen Leerraum von 5 % oder mehr aufweist,

wobei der dichte Teil einen Leerraum von weniger als 5 % aufweist,

wobei der poröse Teil ein Oberflächenteil zum Kontaktieren des weichen Gewebes ist,

wobei der dichte Teil ein Teil nicht zum Kontaktieren des weichen Gewebes ist,

wobei der poröse Teil und der dichte Teil gesinterte Keramikteilchen umfassen und

wobei die medizinische Vorrichtung ein künstliches Produkt ist, das zum Ersetzen defekter Teile von harten Geweben bestimmt ist.

2. Medizinische Vorrichtung nach Anspruch 1,
wobei die arithmetisch gemittelte Rauheit der Oberfläche des dichten Teils weniger als 1,0 $\mu$m beträgt.

3. Medizinische Vorrichtung nach Anspruch 1 oder Anspruch 2,
wobei die Keramikteilchen mindestens eines umfassen ausgewählt aus der Gruppe bestehend aus Zirconiumdioxid, Aluminiumoxid und Lithiumdisilicat.

4. Medizinische Vorrichtung nach einem der Ansprüche 1 bis 3,
wobei der poröse Teil mindestens eines von einer Zelle und einem Wachstumsfaktor umfasst.

5. Medizinische Vorrichtung nach Anspruch 4,
wobei die Zelle mindestens eines umfasst ausgewählt aus der Gruppe bestehend aus einem Gingivalfibroblast, einer Gingivalepithelvorläuferzelle, einem Osteoblast und einem Osteoklast.

6. Medizinische Vorrichtung nach Anspruch 4 oder 5,
wobei der Wachstumsfaktor mindestens einen von einem osteogenen Faktor und einem Zelladhäsionsfaktor umfasst.

7. Medizinische Vorrichtung nach einem der Ansprüche 1 bis 6,
wobei die medizinische Vorrichtung eine Zahnprothese umfasst.

8. Medizinische Vorrichtung nach Anspruch 7,
wobei die Zahnprothese einen künstlichen Zahn umfasst.

9. Verfahren für die Herstellung einer medizinischen Vorrichtung, wobei das Verfahren Folgendes umfasst:

Bilden einer Flüssigkeitsschicht unter Anwendung einer Flüssigkeit, die Keramikteilchen, die einen volumendurchschnittlichen Teilchendurchmesser von weniger als 1 $\mu$m aufweisen, und eine organische Verbindung A umfasst;
Optimieren der Austragsbedingungen, um den porösen Teil und den dichten Teil zu bilden; und
Abgeben einer Härtungsflüssigkeit, die eine organische Verbindung B umfasst, die eine Vernetzungsreaktion mit der organischen Verbindung A aufweist, auf einen vorbestimmten Bereich der Flüssigkeitsschicht,
wobei das Verfahren das Bilden und Abgeben, um ein gehärtetes Produkt herzustellen, und das Sintern des gehärtete Produkts mehrere Male wiederholt, um die medizinische Vorrichtung nach einem der Ansprüche 1 bis 8 herzustellen.

10. Verfahren für die Herstellung einer medizinischen Vorrichtung nach Anspruch 9, wobei das Verfahren ferner das Austragen mindestens eines von einer Zelle und einem Wachstumsfaktor aus einer Tintenstrahldüse zum Abgeben mindestens einer von der Zelle und dem Wachstumsfaktor auf einen vorbestimmten Bereich umfasst.


**Revendications**

1. Dispositif médical comprenant:

une partie poreuse; et

une partie dense,

où une rugosité arithmétique moyenne d'une surface de la partie poreuse est de 2,0 $\mu$m ou plus mais de 10 $\mu$m ou moins,

dans lequel une rugosité arithmétique moyenne d'une surface de la partie dense est inférieure à 2,0 $\mu$m,

dans lequel la partie poreuse présente un niveau de vide de 5 % ou plus,

dans lequel la partie dense présente un niveau de vide inférieur à 5 %,

dans lequel la partie poreuse est une partie de surface pour entrer en contact avec le tissu mou,

dans lequel la partie dense est une partie non en contact avec le tissu mou,

dans lequel la partie poreuse et la partie dense comprennent des particules de céramique frittées, et

dans lequel le dispositif médical est un produit artificiel prévu pour compenser les parties défectueuses des tissus durs.

**2.** Dispositif médical selon la revendication 1,
dans lequel la rugosité arithmétique moyenne de la surface de la partie dense est inférieure à 1,0 $\mu$m.

**3.** Dispositif médical selon la revendication 1 ou la revendication 2,
dans lequel les particules de céramique comprennent au moins l'un sélectionné dans le groupe constitué de l'oxyde de zirconium, de l'alumine, et du disilicate de lithium.

**4.** Dispositif médical selon l'une quelconque des revendications 1 à 3,
dans lequel la partie poreuse comprend au moins n'importe lequel d'une cellule et d'un facteur de croissance.

**5.** Dispositif médical selon la revendication 4,
dans lequel la cellule comprend au moins l'un-e sélectionné-e dans le groupe constitué d'un fibroblaste gingival, d'une cellule progénitrice épithéliale gingivale, d'un ostéoblaste, et d'un ostéoclaste.

**6.** Dispositif médical selon la revendication 4 ou 5,
dans lequel le facteur de croissance comprend au moins n'importe lequel d'un facteur ostéogénique et d'un facteur d'adhésion cellulaire.

**7.** Dispositif médical selon l'une quelconque des revendications 1 à 6,
dans lequel le dispositif médical comprend une prothèse dentaire.

**8.** Dispositif médical selon la revendication 7,
dans lequel la prothèse dentaire comprend une dent artificielle.

**9.** Procédé de production d'un dispositif médical, le procédé comprenant:

la formation d'une couche liquide utilisant un liquide qui comprend des particules de céramique ayant un diamètre de particule moyen en volume inférieur à 1 $\mu$m et un composé organique A;
l'optimisation des conditions de décharge pour former la partie poreuse et la partie dense; et
l'administration d'un liquide de durcissement qui comprend un composé organique B qui présente une réaction de réticulation avec le composé organique A au niveau d'une région prédéterminée de la couche liquide,
le procédé répétant la formation et l'administration une pluralité de fois pour produire un produit durci et le frittage du produit durci pour produire le dispositif médical selon l'une quelconque des revendications 1 à 8.

**10.** Procédé de production d'un dispositif médical selon la revendication 9, le procédé comprenant en outre l'évacuation d'au moins n'importe lequel d'une cellule et d'un facteur de croissance depuis une buse de jet d'encre pour administrer le-la au moins un-e quelconque de la cellule et du facteur de croissance au niveau d'une région prédéterminée.

# FIG. 1

# FIG. 2

**EP 3 216 598 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003531034 W
- JP 2011021218 A
- US 2012315600 A1
- JP 3607300 B
- JP 2000328106 A